# EUROPEAN PATENT APPLICATION

(11) **EP 4 799 623 A1**
(43) Date of publication of application: **02.09.2026**
(21) Application number: 24867567.0
(22) Date of filing: 20.09.2024
(51) Int. Cl.: A61K 31/4965, A61P 21/00

(54) **USE OF LIGUSTRAZINE NITRONE DERIVATIVE IN PREVENTION OR TREATMENT OF MUSCLE STRENGTH REDUCTION OR FUNCTIONAL LOSS CAUSED BY NERVE INJURY**

(30) Priority: 22.09.2023 CN 202311226175
(71) Applicant: Guangzhou Magpie Pharmaceuticals Co., Ltd., Guangzhou, Guangdong 510005 (CN)
(72) Inventor: JING, Mei, Guangzhou, Guangdong 510005 (CN)
(74) Representative: Uexküll & Stolberg
(86) International application number: PCT/CN2024/120138
(87) International publication number: WO 2025/061162

(57) **Abstract**

The present invention provides the use of a ligustrazine nitrone derivative or a pharmaceutically acceptable salt thereof in the preparation of a medicament for preventing or treating muscle strength reduction or functional loss caused by nerve injury; wherein, the ligustrazine nitrone derivative has a structure of the following formula (I): 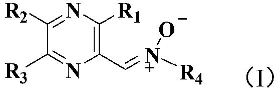 wherein, R₁ and R₃ are each independently C1-C6 alkyl; R₂ is C1-C6 alkyl or 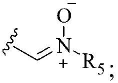 R₄ and R₅ are each independently sec-butyl, isobutyl, tert-butyl, cyclopentyl, or cyclohexyl. Preferably, the C1-C6 alkyl is methyl, ethyl, n-propyl, isopropyl, n-butyl, isobutyl, tert-butyl, or n-pentyl.

## Description

### FIELD OF THE INVENTION

The present invention relates generally to the field of biomedical technology and, more particularly, to the use of a ligustrazine nitrone derivative or a pharmaceutically acceptable salt thereof in the prevention and treatment of muscle strength reduction or functional loss caused by nerve injury.

### BACKGROUND OF THE INVENTION

Muscle strength refers to the strength that one or more muscle groups can produce. Muscle weakness caused by restricted neuromuscular input or impaired signal transmission at the neuromuscular junctions can lead to significant functional loss and increased mortality in motor neuron diseases. Amyotrophic lateral sclerosis (ALS) is a progressive neurological disorder characterized by the deterioration of muscle strength or function due to significant nerve injury. It typically begins with muscle twitching and weakness in the arms or legs, or with speech difficulties. Over three to five years after the onset of symptoms, the muscle ability required to control movement, speech, eating, and breathing gradually decreases, leading to paralysis of the muscles needed for breathing, and ultimately leading to death from respiratory failure. Throughout the entire course of the disease, patients remain fully conscious and retain good skin sensitivity. This condition metaphorically resembles being gradually frozen in place, where individuals can only witness their bodies dying while powerless to intervene. Consequently, ALS is considered "a terminal illness even more cruel than cancer".

Slowing the progression of the disease is crucial, and treatments that improve muscle function performance are also beneficial for patients. However, there are currently no clinically available drugs that can statistically enhance muscle strength in cases of muscle weakness or functional loss caused by nerve injury (e.g., ALS). An article of Mitsumoto H. et al. Revealed that at least 18 drugs have been tested in large phase II or III clinical trials in the past decade, but none demonstrated positive outcomes (Lancet Neurol. 2014 Nov; 13 (11):1127-1138). The primary reason is that current understanding on the pathogenesis of such diseases is very limited. In addition, animal models are often difficult to simulate the true disease state of patients. Many drugs showing efficacy in animal studies have proven ineffective in corresponding clinical trials, such as Coenzyme Q10 (Kaufmann P, Thompson JL, Levy G, Buchsbaum R, Shefner J, Krivickas LS, Katz J, Rollins Y, Barohn RJ, Jackson CE, Tiryaki E, Lomen-Hoerth C, Armon C, Tandan R, Rudnicki SA, Rezania K, Sufit R, Pestronk A, Novella SP, Heiman-Patterson T, Kasarskis EJ, Pioro EP, Montes J, Arbing R, Vecchio D, Barsdorf A, Mitsumoto H, Levin B; QALS Study Group. Phase II trial of CoQ10 for ALS finds insufficient evidence to justify phase III. Ann Neurol. 2009 Aug;66(2):235-44); Levosimendan (Cudkowicz M, Genge A, Maragakis N, Petri S, van den Berg L, Aho VV, Sarapohja T, Kuoppamäki M, Garratt C, Al-Chalabi A; REFALS investigators. Safety and efficacy of oral levosimendan in people with amyotrophic lateral sclerosis (the REFALS study): a randomised, double-blind, placebo-controlled phase 3 trial. Lancet Neurol. 2021 Oct;20(10):821-831); Diprolizumab (Cudkowicz ME, van den Berg LH, Shefner JM, Mitsumoto H, Mora JS, Ludolph A, Hardiman O, Bozik ME, Ingersoll EW, Archibald D, Meyers AL, Dong Y, Farwell WR, Kerr DA; EMPOWER investigators. Dexpramipexole versus placebo for patients with amyotrophic lateral sclerosis (EMPOWER): a randomised, double-blind, phase 3 trial. Lancet Neurol. 2013 Nov;12(11):1059-67); Pioglitazone (Dupuis L, Dengler R, Heneka MT, Meyer T, Zierz S, Kassubek J, Fischer W, Steiner F, Lindauer E, Otto M, Dreyhaupt J, Grehl T, Hermann A, Winkler AS, Bogdahn U, Benecke R, Schrank B, Wessig C, Grosskreutz J, Ludolph AC; GERP ALS Study Group. A randomized, double blind, placebo-controlled trial of pioglitazone in combination with riluzole in amyotrophic lateral sclerosis. PLoS One. 2012;7(6):e37885); Ozaniduzumab (Meininger V, Genge A, van den Berg LH, Robberecht W, Ludolph A, Chio A, Kim SH, Leigh PN, Kiernan MC, Shefner JM, Desnuelle C, Morrison KE, Petri S, Boswell D, Temple J, Mohindra R, Davies M, Bullman J, Rees P, Lavrov A; NOG112264 Study Group. Safety and efficacy of ozanezumab in patients with amyotrophic lateral sclerosis: a randomised, double-blind, placebo-controlled, phase 2 trial. Lancet Neurol. 2017 Mar;16(3):208-216), Vitamin E (Graf M, Ecker D, Horowski R, Kramer B, Riederer P, Gerlach M, Hager C, Ludolph AC, Becker G, Osterhage J, Jost WH, Schrank B, Stein C, Kostopulos P, Lubik S, Wekwerth K, Dengler R, Troeger M, Wuerz A, Hoge A, Schrader C, Schimke N, Krampfl K, Petri S, Zierz S, Eger K, Neudecker S, Traufeller K, Sievert M, Neundörfer B, Hecht M; German vitamin E/ALS Study Group. High dose vitamin E therapy in amyotrophic lateral sclerosis as add-on therapy to riluzole: results of a placebo-controlled double-blind study. J Neural Transm (Vienna). 2005 May;112(5):649-60). The Relevant Chinese Guidelines (2012 edition) and The Chinese Expert Consensus (2022 edition) issued by the Neurology Branch of the Chinese Medical Association clearly indicate that although multiple drugs have shown certain therapeutic effects in animal models in animal experiments, such as Creatine, high-dose Vitamin E, coenzyme Q10, lithium carbonate, Ciliary Neurotrophic Factor, Insulin-like Growth Factor, Lamotrigine, etc., they have not been proven effective in clinical studies (Chinese Journal of Neurology, 2012, 45(7):531-533; Chinese Journal of Neurology, 2022, 55(6):581-588).

### SUMMARY OF THE INVENTION

In order to solve the problems in the existing technology, the present invention provides a use of ligustrazine nitrone derivatives or pharmaceutically acceptable salts thereof in the prevention and treatment of muscle strength reduction or functional loss caused by nerve injury.

The present invention provides the use of ligustrazine nitrone derivatives or pharmaceutically acceptable salts thereof in the prevention and treatment of muscle strength reduction or functional loss caused by nerve injury; wherein, the ligustrazine nitrone derivative has a structure of the following formula (I): 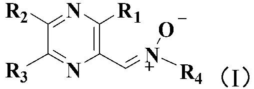 wherein, R₁ and R₃ are each independently C1-C6 alkyl; R₂ is C1-C6 alkyl or 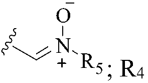 and R₅ are each independently sec-butyl, isobutyl, tert-butyl, cyclopentyl, or cyclohexyl.
Preferably, the C1-C6 alkyl is methyl, ethyl, n-propyl, isopropyl, n-butyl, isobutyl, tert-butyl, or n-pentyl.

According to an embodiment of the present invention, the ligustrazine nitrone derivative has a structure of the following formula, TBN or TN-2:

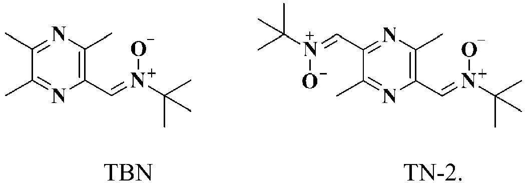

The present invention provides the use for alleviating muscle strength reduction or functional loss related diseases caused by nerve injury, wherein the use comprises administering to a patient in need a therapeutic effective amount of the ligustrazine nitrone derivative (such as TBN or TN-2) or a pharmaceutically acceptable salt thereof.

According to an embodiment of the present invention, the muscle strength reduction or functional loss is caused by Amyotrophic Lateral Sclerosis (ALS).

According to an embodiment of the present invention, the muscle with decreased strength or functional loss includes biceps brachii, triceps brachii, deltoid, pectoralis major, forearm flexor, thenar muscle, hypothenar muscle, tongue muscle, facial muscle, laryngeal muscle, neck muscle, trapezius muscle, sternocleidomastoid muscle, extensor digitorum, first interosseous muscle, abductor pollicis longus, abductor pollicis brevis, extensor digitorum, thoracic paraspinal muscle, respiratory muscle, or a combination thereof..

According to one embodiment of the present invention, the main symptoms of muscle strength reduction or functional loss caused by nerve injury include reduced ability in grip strength, speech, drooling, swallowing, handwriting, cutting food and using utensils, dressing and hygiene, difficulty breathing, sitting breathing, impaired respiratory function, or a combination thereof.

Muscle strength refers to the maximum strength that one or more muscle groups can produce, and the upper limb grip strength has been widely recognized as an evaluation indicator of muscle strength. Grip strength is directly related to the biceps brachii, triceps brachii, deltoid, pectoralis major, forearm flexor, thenar muscle, and hypothenar muscle muscles. In addition, research has confirmed that grip strength is significantly correlated with parameters such as lower limb strength, quadriceps torque, and gastrocnemius muscle cross-sectional area, and is linearly correlated with the ability of daily activity. Grip strength tester is the most commonly used grip strength testing tool.

The ligustrazine nitrone derivative of the present invention can be used to improve, diminish or slow down the degradation of muscle strength (such as grip strength, medulla oblongata function, or respiratory function) caused by nerve injury in subjects. In some embodiments, the use can lead to more significant improvements in upper limb strength in subjects compared to other muscle groups.

Nerve injury may cause a decline in medulla oblongata function, mainly involving such as tongue muscle, trapezius muscle, and sternocleidomastoid muscle, which can cause speech problems and swallowing difficulties, directly affecting the patient's quality of life.

In terms of respiratory function, the scores of forced vital capacity (FVC), dyspnea, orthostatic breathing, and respiratory impairment can be used to monitor the response of patients and/or subjects to the treatment of muscle strength reduction or functional loss caused by nerve injury. The ligustrazine nitrone derivative of the present invention can be used for the treatment of degradation of respiratory muscle strength and/or deterioration of lung function caused by nerve injury. The method provided by the present invention can be used to improve or maintain the respiratory muscle and/or lung function of the subject, or slow down the deterioration of the respiratory muscle and/or lung function of the subject. The respiratory muscle and/or lung function of the subject can be evaluated using any suitable method described herein or known in the art. For example, scores of respiratory function (such as dyspnea, orthostatic breathing, and impaired respiratory) and medulla oblongata function (such as speech, swallowing, and salivation) can be assessed using the ALSFRS-R scale. ALSFRS-R scale is well-know in the art for assessing ALS (rating 0-4), which is used to determine the ability and independence of subjects in their functional activities.

The present invention provides a use of the ligustrazine nitrone derivative or a pharmaceutically acceptable salt thereof in the manufacture of a medicament for the prevention and treatment of muscle strength reduction or functional loss caused by nerve injury, wherein the medicament comprising essentially a therapeutically effective amount of the ligustrazine nitrone derivative is administered orally in unit dose form for preventing, treating, reducing the risk of, delaying the occurrence of, and/or delaying the progression of the muscle strength reduction or functional loss, and the therapeutically effective amount is of about 100-5000 mg, about 300-4000 mg, about 600-3000 mg, or about 1200-2400 mg per day.

According to some embodiments of the present invention, the pharmaceutically acceptable salt is a salt formed by the ligustrazine nitrone derivative and an acid, and the acid is for example hydrochloric acid, hydrobromic acid, methanesulfonic acid, p-toluenesulfonic acid, benzenesulfonic acid, ethanesulfonic acid, phosphoric acid, acetic acid, propionic acid, benzoic acid, hexanoic acid, hydroiodic acid, nitric acid, sulfuric acid, salicylic acid, oxalic acid, tartaric acid, stearic acid, malic acid, maleic acid, fumaric acid, trifluoroacetic acid, cinnamic acid, 2-naphthalenesulfonic acid, succinic acid, D-gluconic acid, dodecylsulfuric acid, pyrosulfuric acid, pyruvic acid, cyclopentanone, citric acid, lactic acid, or aspartic acid.

According to an embodiment of the present invention, the ligustrazine nitrone derivative (such as TBN or TN-2), as an active pharmaceutical component, can be formulated into a pharmaceutical composition (drug formulations) with a pharmaceutically acceptable carrier.

According to an embodiment of the present invention, the pharmaceutical composition is any composition suitable for oral, sublingual, local inhalation (nasal spray), rectal, intramuscular, dermal, subcutaneous or intravenous administration, preferably for oral administration.

As used herein, the term of "a pharmaceutically acceptable carrier" refers to a carrier compatible with other components of the formulation and harmless to its recipient. Examples of pharmaceutically acceptable carriers are known to those skilled in the art.

As appropriate, the medicament is formulated in discrete dosage units and can be prepared by any method well-known in the pharmaceutical field. The method of preparation can include the following steps: mixing the active ingredient with one or more pharmaceutically acceptable carriers, and then shaping the product into a desired dosage form. The pharmaceutical composition can be formulated in the forms of, for example, tablets, granules, fine granules, powders, capsules, small capsules, soft capsules, pills, oral solutions, syrups, chewable tablets, sugar coated tablets, effervescent tablets, drops, suspensions, rapid dissolving tablets, oral rapid dispersing tablets, sustained-release tablets, sustained-release capsules, enteric coated tablets or enteric coated capsules, and so on.

According to an embodiment of the present invention, the medicament for oral administration can be conveniently provided in divided doses to be administered once a day or at appropriate intervals, such as two, three or more doses per day.

Unless otherwise defined, all technical and scientific terms used herein have the same meanings as those understood by ordinary skilled persons in the art. This disclosure describes the methods and materials used in the present invention, while other suitable methods and materials known in this field can also be used. The materials, methods, and examples are illustrative only and not restrictive.

As used herein, the terms "subject" and "patient" are interchangeable. The subject or patient is a human patient or human subject. For the terms "for example" and "such as" and their grammatical equivalents, unless otherwise specified, they should be understood as following the phrase "not limited to" or "and not limited to".

As used herein, the term "therapeutically effective amount" or "pharmaceutically effective dose" refers to the amount of active compound or agent in the human body that triggers the medical response that physicians or other clinical doctors are seeking. The therapeutically or pharmaceutically effective amount of a compound is at least the minimum amount necessary to improve, alleviate, reduce, delay, decrease, relieve, or cure one or more of the symptoms, conditions, or causes of a disease, disorder, or syndrome.

The term 'treatment' includes the therapeutic treatment of patients who have developed the condition in question. Therapeutic treatment can be used to alleviate symptoms of specific indications, or to reverse or partially reverse the condition of indications, or to stop or slow down disease progression. Therefore, the composition and method of the present invention can be used for therapeutic treatment, such as for a period of time, as well as for chronic treatment.

The terms "preventive treatment", "preventive therapy", and "prevention" can be used interchangeably and include the treatment of patients at risk of developing the conditions described above, in order to reduce the risk. As used herein, the term "treatment" also refers to the delay, improvement, or prevention of disease progression (i.e., known or expected disease progression), severity, and/or duration due to the administration of one or more therapies; or delay, improve or prevent the progression of one or more symptoms, clinical manifestations, observations or measurements; or prevent or slow down the negative progression of pathological evaluation (i.e., "manage" rather than "cure" the disease).

### DETAILED DESCRIPTION OF THE EMBODIMENTS

The present invention will be described in detail through specific embodiments. It is pointed out that the following embodiments are only used for further explanation of the present invention and cannot be understood as limiting the scope of the present invention. Unless otherwise specified, the quantities mentioned in this invention are all parts by weight, and the percentages mentioned are all mass percentages. In the embodiments of the present invention, "n" represents the number of subjects participating in the experiment.

### Example 1. Clinical Trial

The present invention adopts a multicenter, randomized, double-blind, placebo-controlled parallel design.

Test subjects: Patients diagnosed with ALS (all signed informed consent forms).

The subjects were randomly assigned (1:1:1) to receive 600 mg, 1200 mg of tetramethylpyrazine nitrone (TBN) or placebo twice daily for 180 days. 148 subjects were included in the FAS set, of which 50 subjects received placebo treatment, 46 subjects received TBN treatment with 600 mg/time (twice a day), and 52 subjects received TBN treatment with 1200 mg/time (twice a day).

An electronic grip strength meter (electronic grip strength tester) is used to test the maximum grip strength of the subject. The experimental results showed that TBN statistically improved the grip strength of the subjects, the baseline of the placebo group was 16.13 kg, and after 180 days of treatment the relative baseline change was -7.60 kg; the baseline of the 600 mg group was 14.53 kg, and after 180 days of treatment the relative baseline change was -6.53 kg, with a 13.95% increase in the rate of change in difference compared to the placebo group; the baseline of the 1200 mg group was 16.51 kg, and after 180 days of treatment the relative baseline change was -5.14 kg, with a 32.37% increase in the rate of change in difference compared to the placebo group (p=0.037).

In addition, the experimental results showed that TBN can also improve the medulla oblongata function and respiratory function of the subjects. Taking the 1200 mg group as an example. In terms of the score of the medulla oblongata function, the baseline for the placebo group was 11.0 points, and after 180 days of treatment the relative baseline change was -1.26 points; the baseline for the 1200 mg group was 11.4 points, and after 180 days of treatment the relative baseline change was -1.12 points, with a 11.11% increase in the rate of change in difference compared to the placebo group. In terms of the score of the respiratory function, the baseline for the placebo group was 12.00 points, and after 180 days of treatment the relative baseline change was -1.21 points; the baseline of the 1200 mg group was 12.00 points, and after 180 days of treatment the relative baseline change was -0.96 points, with a 20.66% increase in the rate of change in difference compared to the placebo group.

In clinical trials, during the 12 week observation period before randomization, all subjects under the age of 65 with a 1-2 point decrease in ALSFRS-R (≥1 and ≤2) after 180 days of treatment had their ALSFRS-R score, forced vital capacity (% FVC), and muscle strength (grip strength) as shown in Table 1, and their scores of the medulla oblongata function and respiratory function as shown in Table 2.

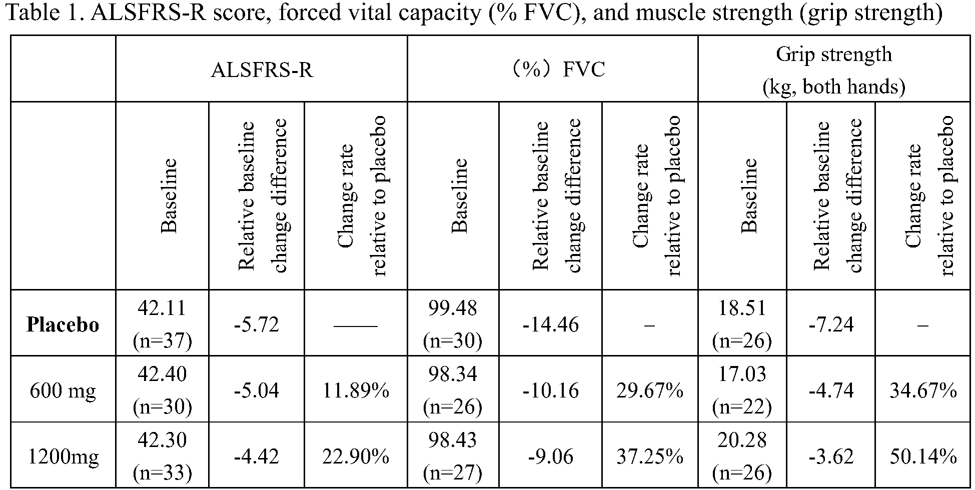

From Table 1, it is evident that TBN significantly improved the ALSFRS-R score, forced vital capacity (% FVC), and muscle strength of the subjects. In terms of ALSFRS-R score, the baseline for the placebo group was 42.11 points, and after 180 days of treatment the relative baseline change was -5.72 points; the baseline score for the 600 mg group was 42.40 points, and after 180 days of treatment the relative baseline change was -5.04 points, indicating an 11.89% increase in the difference rate compared to the placebo group; the baseline for the 1200 mg group was 42.30 points, and after 180 days of treatment the relative baseline change was -4.42 points, with a 22.90% increase in the difference rate compared to the placebo group. In terms of forced vital capacity (FVC), the baseline of the placebo group was 99.48%, and the relative baseline change after 180 days of treatment was -14.46%; the baseline of the 600 mg group was 98.34%, and after 180 days of treatment the relative baseline change was -10.16%, with a difference change rate of 29.67% compared to the placebo group; the baseline of the 1200 mg group was 98.43%, and after 180 days of treatment the relative baseline change was -9.06%, with a 37.25% increase in the difference rate compared to the placebo group. In terms of muscle strength (grip strength), the baseline of the placebo group was 18.51 kg, and the relative baseline change after 180 days of treatment was -7.24 kg; the baseline of the 600 mg group was 17.03 kg, and after 180 days of treatment the relative baseline change was -4.74 kg, with a 34.67% increase in the difference rate compared to the placebo group; the baseline of the 1200 mg group was 20.28 kg, and after 180 days of treatment the relative baseline change was -3.62 kg, with a 50.14% increase in the difference rate compared to the placebo group (p=0.011).

**Table 2. Scores of medulla oblongata function and respiratory function**

| | Score of medulla oblongata function | | | Score of respiratory function | | |
|---|---|---|---|---|---|---|
| | Baseline | Relative baseline change difference | Change rate relative to placebo | Baseline | Relative baseline change difference | Change rate relative to placebo |
| **Placebo (n=37)** | 11.0 | -1.02 | - | 12.00 | -0.71 | - |
| 600 mg **(n=30)** | 11.1 | -0.67 | 35.29% | 12.00 | -0.17 | 76.06% |
| 1200mg **(n=33)** | 11.4 | -0.36 | 64.71% | 12.00 | -0.11 | 85.92% |

From Table 2, it is evident that TBN significantly improved the scores of medulla oblongata and respiratory functions of the subjects. In terms of the medulla oblongata function, the baseline for the placebo group was 11.0 points, and after 180 days of treatment the relative baseline change was -1.02; the baseline for the 600 mg group was 11.1 points, and after 180 days of treatment the relative baseline change was -0.67 points, with a 35.29% increase in the difference rate compared to the placebo group; the baseline for the 1200 mg group was 11.4 points, and after 180 days of treatment the relative baseline change was -0.36 points, with a 64.71% increase in the difference rate compared to the placebo group (p=0.040). In terms of the respiratory function, the placebo group had a baseline score of 12.00 and a relative baseline change of -0.71 after 180 days of treatment; the baseline of the 600 mg group was 12.00 points, and after 180 days of treatment the relative baseline change was -0.17 points, with a 76.06% increase in the difference rate compared to the placebo group (p=0.040); the baseline of the 1200 mg group was 12.00 points, and after 180 days of treatment the relative baseline change was -0.11 points, with an increase of 85.92% in the difference rate compared to the placebo group (p=0.021).

In addition, compared to the placebo group, the subjects' writing ability and ability to use utensils also improved significantly. Taking the 1200 mg group as an example, in terms of writing ability, the baseline for the placebo group was 3.27 points, and the relative baseline change after 180 days of treatment was -0.61 points; the baseline for the 1200 mg group was 3.38 points, and after 180 days of treatment the relative baseline change was -0.39 points, with a 36.07% increase in the difference rate compared to the placebo group. In terms of cutting food and using utensils, the baseline of the placebo group was 3.04 points, and the relative baseline change after 180 days of treatment was -0.94 points; the baseline of the 1200 mg group was 3.04 points, and after 180 days of treatment the relative baseline change was -0.33 points, with a 35.11% increase in the difference rate compared to the placebo group. In terms of clothing and personal hygiene, the baseline for the placebo group was 3.00 points, and the relative baseline change after 180 days of treatment was -0.87 points; the baseline score for the 1200 mg group was 2.92 points, and after 180 days of treatment the relative baseline change was -0.75 points, with a 13.79% increase in the difference rate compared to the placebo group.

## Claims

1. Use of a ligustrazine nitrone derivative or a pharmaceutically acceptable salt thereof in the preparation of a medication for the prevention and treatment of muscle strength reduction or functional loss caused by nerve injury; wherein, the ligustrazine nitrone derivative has a structure of the following formula (I): 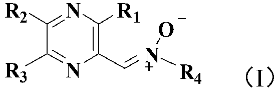 wherein, R₁ and R₃ are each independently C1-C6 alkyl; R₂ is C1-C6 alkyl or 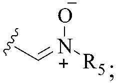 R₄ and R₅ are each independently sec-butyl, isobutyl, tert-butyl, cyclopentyl, or cyclohexyl.

2. The use according to claim 1, wherein the C1-C6 alkyl is methyl, ethyl, n-propyl, isopropyl, n-butyl, isobutyl, tert-butyl, or n-pentyl.

3. The use according to claim 1, wherein the ligustrazine nitrone derivative has a structure of the following formula, TBN or TN-2:

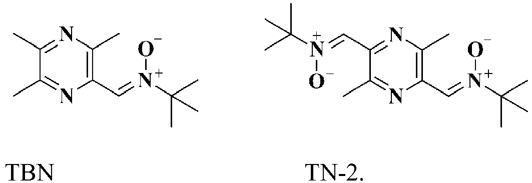

4. The use according to any one of claims 1-3, wherein the muscle strength reduction or functional loss is caused by Amyotrophic Lateral Sclerosis (ALS).

5. The use according to any one of claims 1-3, wherein the muscle with decreased strength or functional loss is selected from biceps brachii, triceps brachii, deltoid, pectoralis major, forearm flexor, thenar muscle, hypothenar muscle, tongue muscle, facial muscle, laryngeal muscle, neck muscle, trapezius muscle, sternocleidomastoid muscle, extensor digitorum, first interosseous muscle, abductor pollicis longus, abductor pollicis brevis, extensor digitorum, thoracic paraspinal muscle, respiratory muscle, or a combination thereof.

6. The use according to any one of claims 1-3, wherein the ligustrazine nitrone derivative can be used to improve the symptoms related to patients' grip strength, speech, drooling, swallowing, handwriting, cutting food and using utensils, dressing and hygiene, dyspnea, orthostatic breathing, impaired respiratory function, or a combination thereof.

7. The use according to any one of claims 1-3, wherein the medication comprises a therapeutically effective amount of the ligustrazine nitrone derivative, and is administered in the form of a unit dose for preventing, treating, reducing the risk of, delaying the occurrence of, and/or delaying the progression of the muscle strength reduction or functional loss.

8. The use according to claim 7, wherein the use comprises administering 100-5000 mg of the ligustrazine nitrone derivative to per day.

9. The use according to any one of claims 1-3, wherein the pharmaceutically acceptable salt is a salt formed by the ligustrazine nitrone derivative and an acid, and the acid is hydrochloric acid, hydrobromic acid, methanesulfonic acid, p-toluenesulfonic acid, benzenesulfonic acid, ethanesulfonic acid, phosphoric acid, acetic acid, propionic acid, benzoic acid, hexanoic acid, hydroiodic acid, nitric acid, sulfuric acid, salicylic acid, oxalic acid, tartaric acid, stearic acid, malic acid, maleic acid, fumaric acid, trifluoroacetic acid, cinnamic acid, 2-naphthalenesulfonic acid, succinic acid, D-gluconic acid, dodecylsulfuric acid, pyrosulfuric acid, pyruvic acid, cyclopentanone, citric acid, lactic acid, or aspartic acid.
